# EUROPEAN PATENT APPLICATION

(11) **EP 3 925 623 A1**
(43) Date of publication of application: **22.12.2021**
(21) Application number: 20754996.5
(22) Date of filing: 13.02.2020
(51) Int. Cl.: A61K 45/00, A61P 1/02, A61P 19/00, A61P 43/00, A61K 31/20, A61K 31/201, A61K 31/202, A61K 31/343

(54) **MEDICINAL COMPOSITION FOR PREVENTING OR TREATING BONE DISEASES**

(30) Priority: 13.02.2019 JP 2019023276
(71) Applicant: Gexval Inc., Fujisawa-shi, Kanagawa 251-0012 (JP)
(72) Inventor: KATO Juran, Fujisawa-shi, Kanagawa 251-0012 (JP); NAITO Takako, Fujisawa-shi, Kanagawa 251-0012 (JP)
(74) Representative: Mössinger, Julia
(86) International application number: PCT/JP2020/005590
(87) International publication number: WO 2020/166662

(57) **Abstract**

The present invention provides a pharmaceutical composition useful for preventing or treating a bone disease associated with an abnormality in the balance between osteoclasts and osteoblasts, particularly periodontal diseases. The pharmaceutical composition for preventing or treating a bone disease according to the present invention contains a GPR40 agonist and a free fatty acid that is an endogenous ligand of GPR40.

## Description

### Technical Field

The present invention relates to a pharmaceutical composition for preventing or treating a bone disease. The present invention also relates to use of a GPR40 agonist.

### Background Art

GPR40, also referred to as free fatty acid receptor 1 (FFAR1), is a G protein-coupled receptor expressed in pancreatic β-cells and bone-related cells (such as osteoclasts, osteoblasts, and precursors thereof) and having a free fatty acid as an endogenous ligand, and the binding of the free fatty acid to GPR40 may regulate the activity of the cell expressing the receptor. For example, pancreatic β-cells are activated through the binding of the free fatty acid to GPR40, which elicits insulin secretion.

Meanwhile, there have been known various GPR40 agonists (agonist drugs) which are compounds capable of binding to GPR40 other than free fatty acids and which are capable of regulating the activity of GPR40-expressing cells. For example, Patent Literature 1 describes a fused-ring compound represented by a specific structural formula as a GPR40 agonist, and further describes in examples that the compound has an activity comparable to that of a free fatty acid (γ-linolenic acid) in the secretion of insulin from pancreatic β-cells, and thus can be used for preventing or treating diabetes mellitus. Non Patent Literature 1 describes a list of various compounds that serve as GPR40 agonists.

One of the fused ring compounds described in Patent Literature 1 is a compound called Fasiglifam (development code: TAK-875), which is [(3S)-6-({2',6'-dimethyl-4'-[3-(methylsulfonyl)propoxy]biphenyl-3-yl}methoxy)-2,3-dihydro-1-benzofuran-3-yl]acetic acid. Non Patent Literature 2 describes that Fasiglifam as an agonist binds to a site (allosteric site) in GPR40 different from the site where a free fatty acid as an endogenous ligand binds, and that Fasiglifam and the free fatty acid when simultaneously acting on GPR40 enhance the secretion of insulin from pancreatic β-cells as compared to when each acting alone.

In Non Patent Literature 3, the manner in which linolenic acid, Fasiglifam (TAK-875), and other GPR40 agonists (GW9508, TUG-770, AMG 837, AM 8182, AM 1638) bind to GPR40 has been analyzed by a molecular structure model. Non Patent Literature 4 also describes in detail the manner in which Fasiglifam (TAK-875) binds to GPR40 by a molecular structure model.

In Non Patent Literature 5, various free fatty acids and a GPR40/GPR120 agonist (GW9508) to GPR40 expressed in osteoclasts and GPR120 expressed in osteoblasts have been researched on their respective independent actions, and it has been reported that these free fatty acids and agonist have an action suppressing the formation of osteoclasts (differentiation and maturation into osteoclasts) caused by the interaction of osteoclasts (RANK) with osteoblasts (RANKL). Non Patent Literature 6 also describes, though a research using the GPR40/GPR120 agonist (GW9508), which the differentiation into osteoclasts is suppressed by the action of GW9508 on GPR40, and thus, bone loss due to ovariectomy can be prevented.

### Citation List

### Patent Literature

Patent Literature 1: WO2008/001931

### Non Patent Literature

Non Patent Literature 1: Yonezawa et al. Free Fatty Acids-Sensing GProtein-Coupled Receptors in Drug Targeting and Therapeutics. Current Medicinal Chemistry, 2013, Vol.20, No.31:3855-3871
Non Patent Literature 2: Yabuki C, Komatsu H, Tsujihata Y, Maeda R, Ito R, et al. (2013) A Novel Antidiabetic Drug, Fasiglifam/TAK-875, Acts as an Ago-Allosteric Modulator of FFAR1. PLoS ONE 8(10): e76280. doi:10.1371/journal.pone.0076280
Non Patent Literature 3: Irina G. Tikhonova and Elena Poerio. Free fatty acid receptors: structuralmodels and elucidation of ligand binding interactions. BMC Structural Biology (2015) 15:16. DOI 10.1186/s12900-015-0044-2
Non Patent Literature 4: Srivastava1 et al. High-resolution structure of the human GPR40 receptor bound to allostericagonist TAK-875. Nature VOL.513, pp. 124-127, September 4, 2014. doi:10.1038/nature13494
Non Patent Literature 5: Cornish et al. Fatty Acids Modulate Osteoclastogenesis. Endocrinology, November 2008, 149(11):5688-5695
Non Patent Literature 6: Wauquier et al. The FreeFatty Acid Receptor G Protein-coupled Receptor 40 (GPR40) Protects from BoneLoss through Inhibition of Osteoclast Differentiation. THE JOURNAL OFBIOLOGICAL CHEMISTRY VOL. 288, NO. 9, pp. 6542-6551, March 1, 2013.

### Summary of Invention

### Technical Problem

Osteoclasts, together with osteoblasts and osteocytes, constitute bone, and the bone is reconstructed through bone resorption by osteoclasts and bone formation by osteoblasts (bone remodeling). Imbalance between the activities of osteoclasts and osteoblasts causes a variety of bone diseases such as osteoporosis and rheumatoid arthritis.

Periodontal diseases caused by periodontopathic bacteria lead to destruction of bone tissue (alveolar bone) as the inflammation in the periodontal tissue increases and becomes chronic, and osteoclasts are involved in the destruction as well. However, it has been reported that bisphosphonates, which have been used as a therapeutic drug for osteoporosis, induce apoptosis in osteoclasts and suppress bone resorption, thereby increasing bone mass, but may cause bisphosphonate-related osteonecrosis of the jaw (BRONJ) in the treatment of periodontal diseases, and it has been also reported that use of an anti-RANKL antibody (denosumab), which is a next-generation therapeutic drug for osteoporosis that inhibits the function of RANKL essential for the formation and differentiation of osteoclasts, also leads to the occurrence of osteonecrosis of the jaw in 1-2%. In order to prevent or treat the periodontal diseases, in which it is necessary to suppress the destruction of the alveolar bone (jawbone) in such a special environment as the oral cavity where the immunity is liable to be imbalanced due to bacterial infection, a new solution has been in demand instead of using the known therapeutic drugs or the like for osteoporosis as they are.

An object of the present invention is to provide a pharmaceutical composition useful for preventing or treating a bone disease associated with an abnormality in the balance between osteoclasts and osteoblasts, particularly periodontal diseases.

### Solution to Problem

The present inventors have revealed that combined use of a GPR40 agonist and a free fatty acid in a culture system of bone marrow-mesenchymal stem cells suppresses differentiation of hematopoietic stem cells into osteoclasts and promotes differentiation of the mesenchymal stem cells into osteoblasts, and have found that these can be excellent active ingredients as a pharmaceutical composition for preventing or treating a bone disease such as periodontal diseases.

That is, the present invention includes the following matters:

### [Item 1]

A pharmaceutical composition for preventing or treating a bone disease, containing a GPR40 agonist and a free fatty acid that is an endogenous ligand of GPR40.

### [Item 2]

The pharmaceutical composition according to item 1, wherein the GPR40 agonist is a compound represented by formula (I) or a salt thereof, or a prodrug of the compound represented by formula (I): 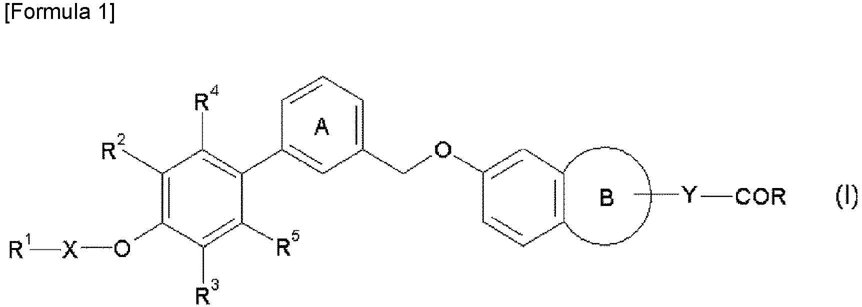 wherein
R¹ represents R⁶-SO₂-(R⁶ represents a substituent) or an optionally substituted 1,1-dioxide tetrahydrothiopyranyl group;
X represents a bond or a divalent hydrocarbon group;
R² and R³ each represent a hydrogen atom, a halogen atom, an optionally substituted hydrocarbon group or an optionally substituted hydroxy group, and are optionally the same or different;
R⁴ and R⁵ each represent a C₁₋₆ alkyl group optionally substituted with a hydroxy group, and are optionally the same or different;
ring A represents a benzene ring that optionally further includes a substituent selected from the group consisting of a halogen atom, an optionally substituted hydrocarbon group, an optionally substituted hydroxy group, and an optionally substituted amino group;
ring B represents a 5- to 7-membered ring;
Y represents a bond or CH₂; and
R represents an optionally substituted hydroxy group.

### [Item 3]

The pharmaceutical composition according to item 1, wherein the free fatty acid that is the endogenous ligand of GPR40 is one or more saturated or unsaturated fatty acids each having 6 to 22 carbon atoms.

### [Item 4]

The pharmaceutical composition according to item 1, wherein the bone disease is alveolar bone resorption in a periodontal disease, alveolar bone resorption after a tooth extraction, bone resorption after alveolar bone reconstruction surgery, or another disease, symptom or condition accompanied by alveolar bone resorption.

### [Item 5]

A method for preventing or treating a bone disease in a mammal, including administering to the mammal an effective dose of a GPR40 agonist and an effective dose of a free fatty acid that is an endogenous ligand of GPR40.

### [Item 6]

Use of a GPR40 agonist and a free fatty acid that is an endogenous ligand of GPR40 for producing a pharmaceutical composition for preventing or treating a bone disease.

### Advantageous Effect of Invention

The present invention provides a pharmaceutical composition for a bone disease that has a preventive or therapeutic effect higher than before. For example, even in the case where the GPR40 agonist alone or the free fatty acid as the ligand of GPR40 alone is not sufficient to suppress the differentiation into osteoclasts, use of the GPR40 agonist and the free fatty acid in combination can achieve a sufficient action and effect; it can be said that these two components when used in combination exhibit a prominent effect as an active ingredient of the pharmaceutical composition for preventing or treating a bone disease. Therefore, the pharmaceutical composition of the present invention makes it possible to effectively prevent or treat alveolar bone loss due to a periodontal disease or the like, which has been difficult so far.

### Brief Description of Drawing

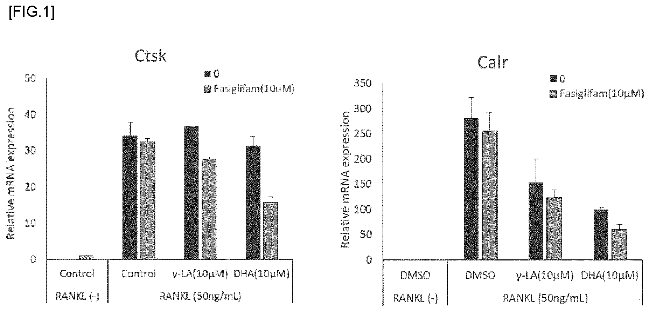

[Figure 1] Figure 1 shows graphs showing the result of expression levels of osteoclast marker genes (Nfatc1, Ctsk, Calcr) measured in Examples.

### Description of Embodiments

### -Pharmaceutical composition-

A pharmaceutical composition of the present invention contains a GPR40 agonist as a first component and a free fatty acid that is an endogenous ligand of GPR40 as a second component, and is used for preventing or treating a bone disease.

### - First component: GPR40 agonist

Various compounds are known as "GPR40 agonists". For example, the compounds described in Patent Literature 1 above and the compounds described in WO2004/041266, WO2004/106276, and WO2005/063729 shown in its background art section, and GW9508, TUG-770, AMG 837, AM 8182, AM 1638 and the like described in Patent Literature 2 can be listed as "GPR40 agonists". The list described in Non Patent Literature 1 can also be referred to. The "GPR40 agonist" that can be used as the first component for the pharmaceutical composition of the present invention is not particularly limited, and can be selected in consideration of the action and effect of the present invention, and any one of the GPR40 agonists may be used alone, or two or more kinds may be used in combination.

The GPR40 agonists include the one that binds to the same site (orthosteric site) as that a free fatty acid binds to in GPR40 and the one that binds to a site (allosteric site) different from that the free fatty acid binds to, and the latter is preferred for the GPR40 agonist used in the present invention.

A medium- or long-chain free fatty acid that is an endogenous ligand of the GPR40 agonist is also an endogenous ligand of GPR120 (FFAR4) in addition to GPR40. The GPR40 agonist used in the present invention may be a non-selective agonist (dual agonist) that also acts as an agonist of GPR120 (acting as an agonist of both GPR40 and GPR120) or a selective agonist that does not act as an agonist of GPR120 (acting only as an agonist of GPR40). For example, Fasiglifam (TAK-875) is a selective agonist for GPR40, and GW9508 is a dual agonist for GPR40 and GPR120.

In a preferred embodiment of the present invention, the GPR40 agonist used as the first component of the pharmaceutical composition of the present invention is a compound represented by formula (I) (referred to herein as "compound (I)") described in Patent Literature 1 or a salt thereof, or a prodrug of compound (I). Fasiglifam (TAK-875) is a representative example of compound (I) and is a suitable compound as the selective agonist of GPR40.

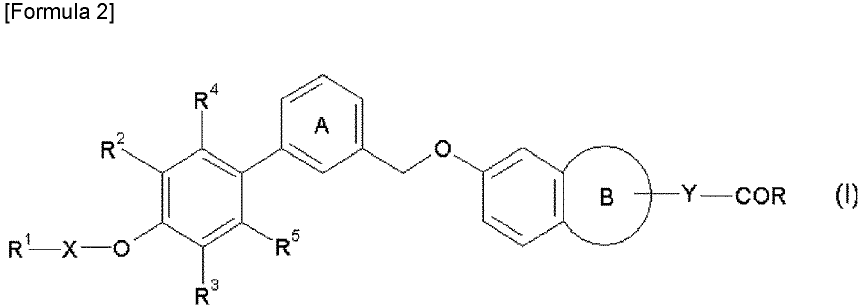

In the formula (I),
R¹ represents R⁶-SO₂- (R⁶ represents a substituent) or an optionally substituted 1,1-dioxide tetrahydrothiopyranyl group;
X represents a bond or a divalent hydrocarbon group;
R² and R³ each represent a hydrogen atom, a halogen atom, an optionally substituted hydrocarbon group or an optionally substituted hydroxy group, and are optionally the same or different;
R⁴ and R⁵ each represent a C₁₋₆ alkyl group optionally substituted with a hydroxy group, and are optionally the same or different;
ring A represents a benzene ring that optionally further includes a substituent selected from the group consisting of a halogen atom, an optionally substituted hydrocarbon group, an optionally substituted hydroxy group, and an optionally substituted amino group;
ring B represents a 5- to 7-membered ring;
Y represents a bond or CH₂; and
R represents an optionally substituted hydroxy group.

Unless otherwise specified, as the "halogen atom" in the present specification, a fluorine atom, a chlorine atom, a bromine atom and an iodine atom can be mentioned.

Unless otherwise specified, as the "optionally substituted hydrocarbon group" in the present specification, for example, an "optionally substituted C₁₋₆ alkyl group", an "optionally substituted C₂₋₆ alkenyl group", an "optionally substituted C₂₋₆ alkynyl group", an "optionally substituted C₃₋₈ cycloalkyl group", an "optionally substituted C₆₋₁₄ aryl group", an "optionally substituted C₇₋₁₆ aralkyl group" and the like can be mentioned.

Unless otherwise specified, as the "C₁₋₆ alkyl group" in the present specification, for example, methyl, ethyl, propyl, isopropyl, butyl, isobutyl, sec-butyl, tert-butyl, pentyl, isopentyl, neopentyl, hexyl and the like can be mentioned.

Unless otherwise specified, as the "C₂₋₆ alkenyl group" in the present specification, for example, vinyl, propenyl, isopropenyl, 2-buten-1-yl, 4-penten-1-yl, 5-hexen-1-yl and the like can be mentioned.

Unless otherwise specified, as the "C₂₋₆ alkynyl group" in the present specification, for example, 2-butyn-1-yl, 4-pentyn-1-yl, 5-hexyn-1-yl and the like can be mentioned.

Unless otherwise specified, as the "C₃₋₈ cycloalkyl group" in the present specification, for example, cyclopropyl, cyclobutyl, cyclopentyl, cyclohexyl and the like can be mentioned.

Unless otherwise specified, as the "C₆₋₁₄ aryl group" in the present specification, for example, phenyl, 1-naphthyl, 2-naphthyl, 2-biphenylyl, 3-biphenylyl, 4-biphenylyl, 2-anthryl and the like can be mentioned. The C₆₋₁₄ aryl may be saturated partially, and as the partially saturated C₆₋₁₄ aryl, for example, tetrahydronaphthyl and the like can be mentioned.

Unless otherwise specified, as the "C₇₋₁₆ aralkyl group" in the present specification, for example, benzyl, phenethyl, diphenylmethyl, 1-naphthylmethyl, 2-naphthylmethyl, 2,2-diphenylethyl, 3-phenylpropyl, 4-phenylbutyl, 5-phenylpentyl, 2-biphenylylmethyl, 3-biphenylylmethyl, 4-biphenylylmethyl and the like can be mentioned.

Unless otherwise specified, as the "optionally substituted hydroxy group" in the present specification, for example, a "hydroxy group", an "optionally substituted C₁₋₆ alkoxy group", an "optionally substituted heterocyclyloxy group", an "optionally substituted C₆₋₁₄ aryloxy group", an "optionally substituted C₇₋₁₆ aralkyloxy group" and the like can be mentioned.

Unless otherwise specified, as the "C₁₋₆ alkoxy group" in the present specification, for example, methoxy, ethoxy, propoxy, isopropoxy, butoxy, isobutoxy, tert-butoxy, pentyloxy, hexyloxy and the like can be mentioned.

Unless otherwise specified, as the "C₁₋₆ alkoxy-C₁₋₆ alkoxy group" in the present specification, for example, methoxymethoxy, methoxyethoxy, ethoxymethoxy, ethoxyethoxy and the like can be mentioned.

As the "heterocyclyloxy group" in the present specification, a hydroxy group substituted with a "heterocyclic group" below can be mentioned. As preferable examples of the heterocyclyloxy group, tetrahydropyranyloxy, thiazolyloxy, pyridyloxy, pyrazolyloxy, oxazolyloxy, thienyloxy, furyloxy and the like can be mentioned.

Unless otherwise specified, as the "C₆₋₁₄ aryloxy group" in the present specification, for example, phenoxy, 1-naphthyloxy, 2-naphthyloxy and the like can be mentioned.

Unless otherwise specified, as the "C₇₋₁₆ aralkyloxy group" in the present specification, for example, benzyloxy, phenethyloxy and the like can be mentioned.

Unless otherwise specified, as the "optionally substituted mercapto group" in the present specification, for example, a "mercapto group", an "optionally substituted C₁₋₆ alkylthio group", an "optionally substituted heterocyclylthio group", an "optionally substituted C₆₋₁₄ arylthio group", an "optionally substituted C₇₋₁₆ aralkylthio group" and the like can be mentioned.

Unless otherwise specified, as the "C₁₋₆ alkylthio group" in the present specification, for example, methylthio, ethylthio, propylthio, isopropylthio, butylthio, sec-butylthio, tert-butylthio and the like can be mentioned.

Unless otherwise specified, as the "heterocyclylthio group" in the present specification, a mercapto group substituted with a "heterocyclic group" below can be mentioned. As preferable examples of the heterocyclylthio group, tetrahydropyranylthio, thiazolylthio, pyridylthio, pyrazolylthio, oxazolylthio, thienylthio, furylthio and the like can be mentioned.

Unless otherwise specified, as the "C₆₋₁₄ arylthio group" in the present specification, for example, phenylthio, 1-naphthylthio, 2-naphthylthio and the like can be mentioned.

Unless otherwise specified, as the "C₇₋₁₆ aralkylthio group" in the present specification, for example, benzylthio, phenethylthio and the like can be mentioned.

Unless otherwise specified, as the "heterocyclic group" in the present specification, for example, a 5- to 14-membered (monocyclic, bicyclic or tricyclic) heterocyclic group containing, as a ring-constituting atom besides carbon atoms, one or two kinds of 1 to 4 hetero atoms selected from a nitrogen atom, a sulfur atom and an oxygen atom, preferably (i) a 5- to 14-membered (preferably 5- to 10-membered) aromatic heterocyclic group, (ii) a 5- to 10-membered non-aromatic heterocyclic group and the like can be mentioned. Of these, a 5- or 6-membered aromatic heterocyclic group is preferable. Specifically, aromatic heterocyclic groups such as thienyl (e.g., 2-thienyl, 3-thienyl), furyl (e.g., 2-furyl, 3-furyl), pyridyl (e.g., 2-pyridyl, 3-pyridyl, 4-pyridyl), thiazolyl (e.g., 2-thiazolyl, 4-thiazolyl, 5-thiazolyl), oxazolyl (e.g., 2-oxazolyl, 4-oxazolyl, 5-oxazolyl), pyrazinyl, pyrimidinyl (e.g., 2-pyrimidinyl, 4-pyrimidinyl), pyrrolyl (e.g., 1-pyrrolyl, 2-pyrrolyl, 3-pyrrolyl), imidazolyl (e.g., 1-imidazolyl, 2-imidazolyl, 4-imidazolyl), pyrazolyl (e.g., 1-pyrazolyl, 3-pyrazolyl, 4-pyrazolyl), triazolyl (e.g., 1-triazolyl, 2-triazolyl), tetrazolyl, pyridazinyl (e.g., 3-pyridazinyl, 4-pyridazinyl), isothiazolyl (e.g., 3-isothiazolyl, 4-isothiazolyl, 5-isothiazolyl), isoxazolyl (e.g., 3-isoxazolyl, 4-isoxazolyl, 5-isoxazolyl), indolyl (e.g., 1-indolyl, 2-indolyl, 3-indolyl), 2-benzothiazolyl, 2-benzoxazolyl, benzimidazolyl (e.g., 1-benzimidazolyl, 2-benzimidazolyl), benzo[b]thienyl (e.g., 2-benzo[b]thienyl, 3-benzo[b] thienyl), benzo[b]furanyl (e.g., 2-benzo[b]furanyl, 3-benzo[b]furanyl), quinolyl (e.g., 2-quinolyl, 3-quinolyl, 4-quinolyl, 5-quinolyl, 8-quinolyl), isoquinolyl (e.g., 1-isoquinolyl, 3-isoquinolyl, 4-isoquinolyl, 5-isoquinolyl) and the like; non-aromatic heterocyclic groups such as pyrrolidinyl (e.g., 1-pyrrolidinyl, 2-pyrrolidinyl, 3-pyrrolidinyl), oxazolidinyl (e.g., 2-oxazolidinyl), imidazolinyl (e.g., 1-imidazolinyl, 2-imidazolinyl, 4-imidazolinyl), piperidinyl (e.g., piperidino, 2-piperidinyl, 3-piperidinyl, 4-piperidinyl), piperazinyl (e.g., 1-piperazinyl, 2-piperazinyl), morpholinyl (e.g., 2-morpholinyl, 3-morpholinyl, morpholino), thiomorpholinyl (e.g., 2-thiomorpholinyl, 3-thiomorpholinyl, thiomorpholino), tetrahydropyranyl and the like, and the like can be mentioned.

Unless otherwise specified, as the "C₁₋₆ alkyl-carbonyl group" in the present specification, for example, acetyl, isobutanoyl, isopentanoyl and the like can be mentioned.

Unless otherwise specified, as the "C₁₋₆ alkoxy-carbonyl group" in the present specification, for example, methoxycarbonyl, ethoxycarbonyl, propoxycarbonyl, tert-butoxycarbonyl and the like can be mentioned.

Unless otherwise specified, as the "C₃₋₈ cycloalkyl-carbonyl group" in the present specification, for example, cyclopentylcarbonyl, cyclohexylcarbonyl and the like can be mentioned.

Unless otherwise specified, as the "C₆₋₁₄ aryl-carbonyl group" in the present specification, for example, benzoyl, 1-naphthoyl, 2-naphthoyl and the like can be mentioned.

Unless otherwise specified, as the "C₇₋₁₆ aralkyl-carbonyl group" in the present specification, for example, phenylacetyl, 2-phenylpropanoyl and the like can be mentioned.

Unless otherwise specified, as the "C₆₋₁₄ aryloxy-carbonyl group" in the present specification, for example, phenoxycarbonyl, naphthyloxycarbonyl and the like can be mentioned.

Unless otherwise specified, as the "C₇₋₁₆ aralkyloxy-carbonyl group" in the present specification, for example, benzyloxycarbonyl, phenethyloxycarbonyl and the like can be mentioned.

Unless otherwise specified, as the "nitrogen-containing heterocyclyl-carbonyl group" in the present specification, for example, pyrrolidinylcarbonyl, piperidinocarbonyl and the like can be mentioned.

Unless otherwise specified, as the "C₁₋₆ alkylsulfonyl group" in the present specification, for example, methylsulfonyl, ethylsulfonyl and the like can be mentioned.

Unless otherwise specified, as the "C₆₋₁₄ arylsulfonyl group" in the present specification, for example, phenylsulfonyl, 1-naphthylsulfonyl, 2-naphthylsulfonyl and the like can be mentioned.

Unless otherwise specified, as the "C₁₋₆ alkylsulfinyl group" in the present specification, for example, methylsulfinyl, ethylsulfinyl and the like can be mentioned.

Unless otherwise specified, as the "C₆₋₁₄ arylsulfinyl group" in the present specification, for example, phenylsulfinyl, 1-naphthylsulfinyl, 2-naphthylsulfinyl and the like can be mentioned.

Unless otherwise specified, as the "optionally esterified carboxyl group" in the present specification, for example, a carboxyl group, a C₁₋₆ alkoxy-carbonyl group, a C₆₋₁₄ aryloxy-carbonyl group, a C₇₋₁₆ aralkyloxy-carbonyl group and the like can be mentioned.

Unless otherwise specified, as the "optionally halogenated C₁₋₆ alkyl group" in the present specification, the above-mentioned "C₁₋₆ alkyl group" optionally substituted with 1 to 5 above-mentioned "halogen atoms" can be mentioned. For example, methyl, ethyl, propyl, isopropyl, butyl, tert-butyl, isobutyl, trifluoromethyl and the like can be mentioned.

Unless otherwise specified, as the "optionally halogenated C₁₋₆ alkoxy group" in the present specification, the above-mentioned "C₁₋₆ alkoxy group" optionally substituted with 1 to 5 above-mentioned "halogen atoms" can be mentioned. For example, methoxy, ethoxy, isopropoxy, tert-butoxy, trifluoromethoxy and the like can be mentioned.

Unless otherwise specified, as the "mono- or di-C₁₋₆ alkyl-amino group" in the present specification, an amino group mono- or di-substituted with the above-mentioned "C₁₋₆ alkyl group(s)" can be mentioned. For example, methylamino, ethylamino, propylamino, dimethylamino, diethylamino and the like can be mentioned.

Unless otherwise specified, as the "mono- or di-C₆₋₁₄ aryl-amino group" in the present specification, an amino group mono- or di-substituted with the above-mentioned "C₆₋₁₄ aryl group(s)" can be mentioned. For example, phenylamino, diphenylamino, 1-naphthylamino, 2-naphthylamino and the like can be mentioned.

Unless otherwise specified, as the "mono- or di-C₇₋₁₆ aralkyl-amino group" in the present specification, an amino group mono- or di-substituted with the above-mentioned "C₇₋₁₆ aralkyl group(s)" can be mentioned. For example, benzylamino, phenethylamino and the like can be mentioned.

Unless otherwise specified, as the "N-C₁₋₆ alkyl-N-C₆₋₁₄ aryl-amino group" in the present specification, an amino group substituted with the above-mentioned "C₁₋₆ alkyl group" and the above-mentioned "C₆₋₁₄ aryl group" can be mentioned. For example, N-methyl-N-phenylamino, N-ethyl-N-phenylamino and the like can be mentioned.

Unless otherwise specified, as the "N-C₁₋₆ alkyl-N-C₇₋₁₆, aralkyl-amino group" in the present specification, an amino group substituted with the above-mentioned "C₁₋₆ alkyl group" and the above-mentioned "C₇₋₁₆ aralkyl group" can be mentioned. For example, N-methyl-N-benzylamino, N-ethyl-N-benzylamino and the like can be mentioned.

Unless otherwise specified, as the "mono- or di-C₁₋₆ alkyl-carbamoyl group" in the present specification, a carbamoyl group mono- or di-substituted with the above-mentioned "C₁₋₆ alkyl group(s)" can be mentioned. For example, methylcarbamoyl, ethylcarbamoyl, dimethylcarbamoyl, diethylcarbamoyl, ethylmethylcarbamoyl and the like can be mentioned.

Unless otherwise specified, as the "mono- or di-C₆₋₁₄ aryl-carbamoyl group" in the present specification, a carbamoyl group mono- or di-substituted with the above-mentioned "C₆₋₁₄ aryl group(s)" can be mentioned. For example, phenylcarbamoyl, 1-naphthylcarbamoyl, 2-naphthylcarbamoyl and the like can be mentioned.

Unless otherwise specified, as the "mono- or di-C₃₋₈ cycloalkyl-carbamoyl group" in the present specification, a carbamoyl group mono- or di-substituted with the above-mentioned "C₃₋₈ cycloalkyl group(s)" can be mentioned. For example, cyclopropylcarbamoyl and the like can be mentioned.

Unless otherwise specified, as the "mono- or di-C₇₋₁₆ aralkyl-carbamoyl group" in the present specification, a carbamoyl group mono- or di-substituted with the above-mentioned "C₇₋₁₆ aralkyl group(s)" can be mentioned. For example, benzylcarbamoyl and the like can be mentioned.

Unless otherwise specified, as the "mono- or di-5- to 7-membered heterocyclyl-carbamoyl group" in the present specification, a carbamoyl group mono- or di-substituted with 5-to 7-membered heterocyclic group(s) can be mentioned. As the 5- to 7-membered heterocyclic group, a heterocyclic group containing, as a ring-constituting atom besides carbon atoms, one or two kinds of 1 to 4 hetero atoms selected from a nitrogen atom, a sulfur atom and an oxygen atom can be mentioned. As preferable examples of the "mono- or di-5 to 7-membered heterocyclyl-carbamoyl group", 2-pyridylcarbamoyl, 3-pyridylcarbamoyl, 4-pyridylcarbamoyl, 2-thienylcarbamoyl, 3-thienylcarbamoyl and the like can be mentioned.

Unless otherwise specified, as the "mono- or di-C₁₋₆ alkyl-sulfamoyl group" in the present specification, a sulfamoyl group mono- or di-substituted with the above-mentioned "C₁₋₆ alkyl group(s)" can be used, for example, methylsulfamoyl, ethylsulfamoyl, dimethylsulfamoyl, diethylsulfamoyl and the like can be mentioned.

Unless otherwise specified, as the "mono- or di-C₆₋₁₄ aryl-sulfamoyl group" in the present specification, a sulfamoyl group mono-, or di-substituted with the above-mentioned "C₆₋₁₄ aryl group(s)" can be used, for example, phenylsulfamoyl, diphenylsulfamoyl, 1-naphthylsulfamoyl, 2-naphthylsulfamoyl and the like can be mentioned.

Unless otherwise specified, as the "mono- or di-C₇₋₁₆ aralkyl-sulfamoyl group" in the present specification, a sulfamoyl group mono- or di-substituted with the above-mentioned "C₇₋₁₆ aralkyl group(s)" can be used, for example, benzylsulfamoyl and the like can be mentioned.

Unless otherwise specified, as the "optionally substituted C₁₋₆ alkyl group", "optionally substituted C₂₋₆ alkenyl group", "optionally substituted C₂₋₆ alkynyl group", "optionally substituted C₁₋₆ alkoxy group" and "optionally substituted C₁₋₆ alkylthio group" in the present specification, for example, a "C₁₋₆ alkyl group", a "C₂₋₆ alkenyl group", a "C₂₋₆ alkynyl group", a "C₁₋₆ alkoxy group" and a "C₁₋₆ alkylthio group", each of which optionally has 1 to 5 substituents at substitutable positions selected from
(1) a halogen atom;
(2) a hydroxy group;
(3) an amino group;
(4) a nitro group;
(5) a cyano group;
(6) a heterocyclic group (preferably furyl, pyridyl, thienyl, pyrazolyl, thiazolyl, oxazolyl) optionally substituted with 1 to 3 substituents selected from a halogen atom, a hydroxy group, an amino group, a nitro group, a cyano group, an optionally halogenated C₁₋₆ alkyl group, a mono- or di-C₁₋₆ alkyl-amino group, a C₆₋₁₄ aryl group, a mono- or di-C₆₋₁₄ aryl-amino group, a C₃₋₈ cycloalkyl group, a C₁₋₆ alkoxy group, a C₁₋₆ alkoxy-C₁₋₆ alkoxy group, a C₁₋₆ alkylthio group, a C₁₋₆ alkylsulfinyl group, a C₁₋₆ alkylsulfonyl group, an optionally esterified carboxyl group, a carbamoyl group, a thiocarbamoyl group, a mono- or di-C₁₋₆ alkyl-carbamoyl group, a mono- or di-C₆₋₁₄ aryl-carbamoyl group, a sulfamoyl group, a mono- or di-C₁₋₆ alkyl-sulfamoyl group and a mono- or di-C₆₋₁₄ aryl-sulfamoyl group;
(7) a mono- or di-C₁₋₆ alkyl-amino group;
(8) a mono- or di-C₆₋₁₄ aryl-amino group;
(9) a mono- or di-C₇₋₁₆ aralkyl-amino group;
(10) an N-C₁₋₆ alkyl-N-C₆₋₁₄ aryl-amino group;
(11) an N-C₁₋₆ alkyl-N-C₇₋₁₆ aralkyl-amino group;
(12) a C₃₋₈ cycloalkyl group;
(13) an optionally halogenated C₁₋₆ alkoxy group;
(14) a C₁₋₆ alkylthio group;
(15) a C₁₋₆ alkylsulfinyl group;
(16) a C₁₋₆ alkylsulfonyl group;
(17) an optionally esterified carboxyl group;
(18) a C₁₋₆ alkyl-carbonyl group;
(19) a C₃₋₈ cycloalkyl-carbonyl group;
(20) a C₆₋₁₄ aryl-carbonyl group;
(21) a carbamoyl group;
(22) a thiocarbamoyl group;
(23) a mono- or di-C₁₋₆ alkyl-carbamoyl group;
(24) a mono- or di-C₆₋₁₄ aryl-carbamoyl group;
(25) a mono- or di-5- to 7-membered heterocyclyl-carbamoyl group;
(26) a C₁₋₆ alkyl-carbonylamino group (e.g., acetylamino, propionylamino) optionally substituted with carboxyl group(s);
(27) a C₆₋₁₄ aryloxy group optionally substituted with 1 to 3 substituents selected from a halogen atom, a hydroxy group, an amino group, a nitro group, a cyano group, an optionally halogenated C₁₋₆ alkyl group, a mono- or di-C₁₋₆ alkyl-amino group, a C₆₋₁₄ aryl group, a mono-or di-C₆₋₁₄ aryl-amino group, a C₃₋₈ cycloalkyl group, a C₁₋₆ alkoxy group, a C₁₋₆ alkoxy-C₁₋₆ alkoxy group, a C₁₋₆ alkylthio group, a C₁₋₆ alkylsulfinyl group, a C₁₋₆ alkylsulfonyl group, an optionally esterified carboxyl group, a carbamoyl group, a thiocarbamoyl group, a mono- or di-C₁₋₆ alkyl-carbamoyl group, a mono- or di-C₆₋₁₄ aryl-carbamoyl group, a sulfamoyl group, a mono- or di-C₁₋₆ alkyl-sulfamoyl group and a mono- or di-C₆₋₁₄ aryl-sulfamoyl group;
(28) a C₆₋₁₄ aryl group optionally substituted with 1 to 3 substituents selected from a halogen atom, a hydroxy group, an amino group, a nitro group, a cyano group, an optionally halogenated C₁₋₆ alkyl group, a mono- or di-C₁₋₆ alkyl-amino group, a C₆₋₁₄ aryl group, a mono-or di-C₆₋₁₄ aryl-amino group, a C₃₋₈ cycloalkyl group, a C₁₋₆ alkoxy group, a C₁₋₆ alkoxy-C₁₋₆ alkoxy group, a C₁₋₆ alkylthio group, a C₁₋₆ alkylsulfinyl group, a C₁₋₆ alkylsulfonyl group, an optionally esterified carboxyl group, a carbamoyl group, a thiocarbamoyl group, a mono- or di-C₁₋₆ alkyl-carbamoyl group, a mono- or di-C₆₋₁₄ aryl-carbamoyl group, a sulfamoyl group, a mono- or di-C₁₋₆ alkyl-sulfamoyl group and a mono- or di-C₆₋₁₄ aryl-sulfamoyl group;
(29) a heterocyclyloxy group;
(30) a sulfamoyl group;
(31) a mono- or di-C₁₋₆ alkyl-sulfamoyl group;
(32) a mono- or di-C₆₋₁₄ aryl-sulfamoyl group;
(33) a C₇₋₁₆ aralkyloxy group optionally substituted with 1 to 3 substituents selected- from a halogen atom, a hydroxy group, an amino group, a nitro group, a cyano group, an optionally halogenated C₁₋₆ alkyl group, a mono- or di-C₁₋₆ alkyl-amino group, a C₆₋₁₄ aryl group, a mono-or di-C₆₋₁₄ aryl-aminb group, a C₃₋₈ cycloalkyl group, a C₁₋₆ alkoxy group, a C₁₋₆ alkoxy-C₁₋₆ alkoxy group, a C₁₋₆ alkylthio group, a C₁₋₆ alkylsulfinyl group, a C₁₋₆ alkylsulfonyl group, an optionally esterified carboxyl group, a carbamoyl group, a thiocarbamoyl group, a mono- or di-C₁₋₆ alkyl-carbamoyl group, a mono- or di-C₆₋₁₄ aryl-carbamoyl group, a sulfamoyl group, a mono- or di-C₁₋₆ alkyl-sulfamoyl group and a mono- or di-C₆₋₁₄ aryl-sulfamoyl group; and the like, can be mentioned.

As the "optionally substituted C₃₋₈ cycloalkyl group", "optionally substituted C₆₋₁₄ aryl group", "optionally substituted C₇₋₁₆ aralkyl group", "optionally substituted heterocyclic group", "optionally substituted heterocyclyloxy group", "optionally substituted C₆₋₁₄ aryloxy group", "optionally substituted C₇₋₁₆ aralkyloxy group", "optionally substituted heterocyclylthio group", "optionally substituted C₆₋₁₄ arylthio group" and "optionally substituted C₇₋₁₆ aralkylthio group" in the present specification, for example, a "C₃₋₈ cycloalkyl group", a "C₆₋₁₄ aryl group", a "C₇₋₁₆ aralkyl group", a "heterocyclic group", a "heterocyclyloxy group", a "C₆₋₁₄ aryloxy group", a "C₇₋₁₆ aralkyloxy group", a "heterocyclylthio group", a "C₆₋₁₄ arylthio group" and a "C₇₋₁₆ aralkylthio group", each of which optionally has 1 to 5 substituents at substitutable positions selected from
(1) a halogen atom;
(2) a hydroxy group;
(3) an amino group;
(4) a nitro group;
(5) a cyano group;
(6) an optionally substituted C₁₋₆ alkyl group;
(7) an optionally substituted C₂₋₆ alkenyl group;
(8) an optionally substituted C₂₋₆ alkynyl group;
(9) a C₆₋₁₄ aryl group optionally substituted with 1 to 3 substituents selected from a halogen atom, a hydroxy group, an amino group, a nitro group, a cyano group, an optionally halogenated C₁₋₆ alkyl group, a mono- or di-C₁₋₆ alkyl-amino group, a C₆₋₁₄ aryl group, a mono-or di-C₆₋₁₄ aryl-amino group, a C₃₋₈ cycloalkyl group, a C₁₋₆ alkoxy group, a C₁₋₆ alkoxy-C₁₋₆ alkoxy group, a C₁₋₆ alkylthio group, a C₁₋₆ alkylsulfinyl group, a C₁₋₆ alkylsulfonyl group, an optionally esterified carboxyl group, a carbamoyl group, a thiocarbamoyl group, a mono- or di-C₁₋₆ alkyl-carbamoyl group, a mono- or di-C₆₋₁₄ aryl-carbamoyl group, a sulfamoyl group, a mono- or di-C₁₋₆ alkyl-sulfamoyl group and a mono- or di-C₆₋₁₄ aryl-sulfamoyl group;
(10) a C₆₋₁₄ aryloxy group optionally substituted with 1 to 3 substituents selected from a halogen atom, a hydroxy group, an amino group, a nitro group, a cyano group, an optionally halogenated C₁₋₆ alkyl group, a mono- or di-C₁₋₆ alkyl-amino group, a C₆₋₁₄ aryl group, a mono-or di-C₆₋₁₄ aryl-amino group, a C₃₋₈ cycloalkyl group, a C₁₋₆ alkoxy group, a C₁₋₆ alkoxy-C₁₋₆ alkoxy group, a C₁₋₆ alkylthio group, a C₁₋₆ alkylsulfinyl group, a C₁₋₆ alkylsulfonyl group, an optionally esterified carboxyl group, a carbamoyl group, a thiocarbamoyl group, a mono- or di-C₁₋₆ alkyl-carbamoyl group, a mono- or di-C₆₋₁₄ aryl-carbamoyl group, a sulfamoyl group, a mono- or di-C₁₋₆ alkyl-sulfamoyl group and a mono- or di-C₆₋₁₄ aryl-sulfamoyl group;
(11) a C₇₋₁₆ aralkyloxy group optionally substituted with 1 to 3 substituents selected from a halogen atom, a hydroxy group, an amino group, a nitro group, a cyano group, an optionally halogenated C₁₋₆ alkyl group, a mono- or di-C₁₋₆ alkyl-amino group, a C₆₋₁₄ aryl group, a mono-or di-C₆₋₁₄ aryl-amino group, a C₃₋₈ cycloalkyl group, a C₁₋₆ alkoxy group, a C₁₋₆ alkoxy-C₁₋₆ alkoxy group, a C₁₋₆ alkylthio group, a C₁₋₆ alkylsulfinyl group, a C₁₋₆ alkylsulfonyl group, an optionally esterified carboxyl group, a carbamoyl group, a thiocarbamoyl group, a mono- or di-C₁₋₆ alkyl-carbamoyl group, a mono- or di-C₆₋₁₄ aryl-carbamoyl group, a sulfamoyl group, a mono- or di-C₁₋₆ alkyl-sulfamoyl group and a mono- or di-C₆₋₁₄ aryl-sulfamoyl group;
(12) a heterocyclic group (preferably furyl, pyridyl, thienyl, pyrazolyl, thiazolyl, oxazolyl) optionally substituted with 1 to 3 substituents selected from a halogen atom, a hydroxy group, an amino group, a nitro group, a cyano group, an optionally halogenated C₁₋₆ alkyl group, a mono- or di-C₁₋₆ alkyl-amino group, a C₆₋₁₄ aryl group, a mono- or di-C₆₋₁₄ aryl-amino group, a C₃₋₈ cycloalkyl group, a C₁₋₆ alkoxy group, a C₁₋₆ alkoxy-C₁₋₆ alkoxy group, a C₁₋₆ alkylthio group, a C₁₋₆ alkylsulfinyl group, a C₁₋₆ alkylsulfonyl group, an optionally esterified carboxyl group, a carbamoyl group, a thiocarbamoyl group, a mono- or di-C₁₋₆ alkyl-carbamoyl group, a mono- or di-C₆₋₁₄ aryl-carbamoyl group, a sulfamoyl group, a mono- or di-C₁₋₆ alkyl-sulfamoyl group and a mono- or di-C₆₋₁₄ aryl-sulfamoyl group;
(13) a mono- or di-C₁₋₆ alkyl-amino group;
(14) a mono- or di-C₆₋₁₄ aryl-amino group;
(15) a mono- or di-C₇₋₁₆ aralkyl-amino group;
(16) an N-C₁₋₆ alkyl-N-C₆₋₁₄ aryl-amino group;
(17) an N-C₁₋₆ alkyl-N-C₇₋₁₆ aralkyl-amino group;
(18) a C₃₋₈ cycloalkyl group;
(19) an optionally substituted C₁₋₆ alkoxy group;
(20) an optionally substituted C₁₋₆ alkylthio group;
(21) a C₁₋₆ alkylsulfinyl group;
(22) a C₁₋₆ alkylsulfonyl group;
(23) an optionally esterified carboxyl group;
(24) a C₁₋₆ alkyl-carbonyl group;
(25) a C₃₋₈ cycloalkyl-carbonyl group;
(26) a C₆₋₁₄ aryl-carbonyl group;
(27) a carbamoyl group;
(28) a thiocarbamoyl group;
(29) a mono- or di-C₁₋₆ alkyl-carbamoyl group;
(30) a mono- or di-C₆₋₁₄ aryl-carbamoyl group;
(31) a mono- or di-5- to 7-membered heterocyclyl-carbamoyl group;
(32) a sulfamoyl group;
(33) a mono- or di-C₁₋₆ alkyl-sulfamoyl group;
(34) a mono- or di-C₆₋₁₄ aryl-sulfamoyl group;
(35) a C₁₋₆ alkyl-carbonylamino group (e.g., acetylamino, propionylamino) optionally substituted with carboxyl group(s);
(36) a heterocyclyloxy group;
and the like, can be mentioned.

Unless otherwise specified, as the "optionally substituted amino group" in the present specification, an amino group optionally substituted with 1 or 2 substituents selected from
(1) an optionally substituted C₁₋₆ alkyl group;
(2) an optionally substituted C₂₋₆ alkenyl group;
(3) an optionally substituted C₂₋₆ alkynyl group;
(4) an optionally substituted C₃₋₈ cycloalkyl group;
(5) an optionally substituted C₆₋₁₄ aryl group;
(6) an optionally substituted C₁₋₆ alkoxy group;
(7) an optionally substituted acyl group;
(8) an optionally substituted heterocyclic group (preferably furyl, pyridyl, thienyl, pyrazolyl, thiazolyl, oxazolyl);
(9) a sulfamoyl group;
(10) a mono- or di-C₁₋₆ alkyl-sulfamoyl group;
(11) a mono- or di-C₆₋₁₄ aryl-sulfamoyl group; and the like, can be mentioned. When the "optionally substituted amino group" is an amino group substituted with 2 substituents, these substituents may form a nitrogen-containing heterocycle together with the adjacent nitrogen atom. As the "nitrogen-containing heterocycle", for example, a 5- to 7-membered nitrogen-containing heterocycle containing, as a ring-constituting atom besides carbon atoms, at least one nitrogen atom and optionally further containing 1 or 2 hetero atoms selected from an oxygen atom, a sulfur atom and a nitrogen atom can be mentioned. As preferable examples of the nitrogen-containing heterocycle, pyrrolidine, imidazolidine, pyrazolidine, piperidine, piperazine, morpholine, thiomorpholine, thiazolidine, oxazolidine and the like can be mentioned.

Unless otherwise specified, as the "optionally substituted acyl group" in the present specification, groups represented by the formula: -COR₇, -CO-OR₇, -SO₂R₇, -SOR₇, - PO(OR₇)(OR₈), -CO-NR₇ₐR₈ₐ and -CS-NR₇ₐR₈ₐ, wherein R₇ and R₈ are the same or different and each is a hydrogen atom, an optionally substituted hydrocarbon group or an optionally substituted heterocyclic group, and R₇ₐ and R₈ₐ are the same or different and each is a hydrogen atom, an optionally substituted hydrocarbon group or an optionally substituted heterocyclic group, or R₇ₐ and R₈ₐ may form an optionally substituted nitrogen-containing heterocycle together with the adjacent nitrogen atom, and the like can be mentioned.

As the "nitrogen-containing heterocycle" of the "optionally substituted nitrogen-containing heterocycle" which R₇ₐ and R₈ₐ form together with the adjacent nitrogen atom, for example, a 5- to 7-membered nitrogen-containing heterocycle containing, as a ring-constituting atom besides carbon atoms, at least one nitrogen atom and optionally further containing 1 to 2 hetero atoms selected from an oxygen atom, a sulfur atom and a nitrogen atom can be mentioned. As preferable examples of the "nitrogen-containing heterocycle", pyrrolidine, imidazolidine, pyrazolidine, piperidine, piperazine, morpholine, thiomorpholine, thiazolidine, oxazolidine and the like can be mentioned.

The nitrogen-containing heterocycle optionally has 1 to 2 substituents at substitutable positions. As these substituents, a hydroxy group, an optionally halogenated C₁₋₆ alkyl group, a C₆₋₁₄ aryl group, a C₇₋₁₆ aralkyl group and the like can be mentioned.

As preferable examples of the "optionally substituted acyl group",
a formyl group;
a carboxyl group;
a carbamoyl group;
a C₁₋₆ alkyl-carbonyl group;
a C₁₋₆ alkoxy-carbonyl group;
a C₃₋₈ cycloalkyl-carbonyl group;
a C₆₋₁₄ aryl-carbonyl group;
a C₇₋₁₆ aralkyl-carbonyl group;
a C₆₋₁₄ aryloxy-carbonyl group;
a C₇₋₁₆ aralkyloxy-carbonyl group;
a mono- or di-C₁₋₆ alkyl-carbamoyl group;
a mono- or di-C₆₋₁₄ aryl-carbamoyl group;
a mono- or di-C₃₋₈ cycloalkyl-carbamoyl group;
a mono- or di-C₇₋₁₆ aralkyl-carbamoyl group;
a C₁₋₆ alkylsulfonyl group;
a C₆₋₁₄ arylsulfonyl group optionally substituted with nitro group(s);
a nitrogen-containing heterocyclyl-carbonyl group;
a C₁₋₆ alkylsulfinyl group;
a C₆₋₁₄ arylsulfinyl group;
a thiocarbamoyl group;
a sulfamoyl group;
a mono- or di-C₁₋₆ alkyl-sulfamoyl group;
a mono- or di-C₆₋₁₄ aryl-sulfamoyl group;
a mono- or di-C₇₋₁₆ aralkyl-sulfamoyl group;
and the like can be mentioned.

Each symbol in the formula (I) is described in detail in the following.

R₁ is R₆-SO₂- (wherein R₆ is a substituent) or an optionally substituted 1,1-dioxidotetrahydrothiopyranyl group.

As used herein, as the "substituent" for R₆, an "optionally substituted hydrocarbon group", an "optionally substituted heterocyclic group", an "optionally substituted hydroxy group", an "optionally substituted amino group", an "optionally substituted mercapto group", a "cyano group", an "optionally substituted acyl group", a "halogen atom" and the like can be mentioned.

R₆ is preferably an optionally substituted hydrocarbon group, more preferably a C₁₋₆ alkyl group (preferably methyl, ethyl).

The "1,1-dioxidotetrahydrothiopyranyl group" of the "optionally substituted 1,1-dioxidotetrahydrothiopyranyl group" for R₁ optionally has 1 to 5 substituents, preferably 1 to 3, substituents at substitutable positions. As the "substituent", those exemplified as the substituents of the aforementioned "optionally substituted C₃₋₈ cycloalkyl group" can be used. When the "1,1-dioxidotetrahydrothiopyranyl group" has two or more substituents, respective substituents may be the same or different.

The "substituent" is preferably a hydroxy group and the like.

R₁ is preferably a C₁₋₆ alkylsulfonyl group (preferably methylsulfonyl, ethylsulfonyl) or a 1,1-dioxidotetrahydrothiopyranyl group, each of which is optionally substituted with 1 to 3 substituents selected from a hydroxy group and the like, more preferably a C₁₋₆ alkylsulfonyl group (preferably methylsulfonyl, ethylsulfonyl), or a 1,1-dioxidotetrahydrothiopyranyl group optionally substituted with hydroxy group(s).

As another embodiment, R¹ is preferably R₆-SO₂- (wherein R₆ is a substituent), more preferably a C₁₋₆ alkylsulfonyl group (preferably methylsulfonyl, ethylsulfonyl).

X is a bond or a divalent hydrocarbon group.

As the "divalent hydrocarbon group" for X, for example, a divalent chain hydrocarbon group, a divalent cyclic
hydrocarbon group, a divalent chain-cyclic hydrocarbon group can be mentioned.

### Specifically,

(1) a C₁₋₁₀ alkylene group (e.g., -CH₂-, -(CH₂)₂-, -(CH₂)₃-, -(CH₂)₄-, -(CH₂)₅-, -(CH₂)₆-, -CHCH₃-, -C(CH₃)₂-, -(CH(CH₃))₂-, -(CH₂)₂C(CH₃)₂-, -(CH₂)₃C(CH₃)₂-);
(2) a C₂₋₁₀ alkenylene group (e.g., -CH=CH-, -CH₂-CH=CH-, -CH=CH-CH₂-, -CH=CH-CH₂-CH₂-, -C(CH₃)₂-CH=CH-, -CH₂-CH=CH-CH₂-, -CH₂-CH₂-CH=CH-, -CH=CH-CH=CH-, -CH=CH-CH₂-CH₂-CH₂-);
(3) a C₂₋₁₀ alkynylene group (e.g., -C=C-, -CH₂-C≡C-, -CH₂-C≡C-CH₂-CH₂-);
(4) a C₃₋₈ cycloalkylene group (e.g., 1,2-cyclopropylene, 1,3-cyclobutylene, 1,3-cyclopentylene, 1,3-cyclohexylene, 1,4-cyclohexylene, 1,4-cycloheptylene, 1,5-cyclooctylene);
(5) a C₆₋₁₄ arylene group (e.g., phenylene (e.g., 1,2-phenylene, 1,3-phenylene, 1,4-phenylene), naphthylene (e.g., 1,2-naphthylene, 1,3-naphthylene, 1,4-naphthylene, 1,5-naphthylene, 1,6-naphthylene, 1,7-naphthylene, 1,8-naphthylene, 2,3-naphthylene, 2,6-naphthylene, 2,7-naphthylene), biphenylene (e.g., 2,2'-biphenylene, 3,3'-biphenylene, 4,4'-biphenylene) and the like. The C₆₋₁₄ arylene may be saturated partially, and as the partially saturated C₆₋₁₄ arylene, for example, tetrahydronaphthylene and the like can be mentioned);
(6) a combination of any two selected from the above-mentioned (1) to (5) (e.g., methylene-phenylene, phenylene-methylene, ethylene-phenylene, phenylene-ethylene, methylene-cyclohexylene, cyclohexylene-methylene, methylene-naphthylene, naphthylene-methylene); and the like can be mentioned.

X is preferably a bond or a C₁₋₁₀ alkylene group (preferably a C₁₋₆ alkylene group, more preferably a straight chain C₁₋₃ alkylene group), more preferably a C₁₋₆ alkylene group (preferably a straight chain C₁₋₃ alkylene group, more preferably -(CH₂)₃-).

R₂ and R₃ are the same or different and each is a hydrogen atom, a halogen atom, an optionally substituted hydrocarbon group or an optionally substituted hydroxy group.

Preferably, R₂ and R₃ are the same or different and each is
a hydrogen atom;
a halogen atom; or
a C₁₋₆ alkyl group (preferably methyl),
and more preferably, R₂ and R₃ are each a hydrogen atom.

R₄ and R₅ are the same or different and each is a C₁₋₆ alkyl group optionally substituted with hydroxy group(s). Preferably, R₄ and R₅ are the same or different and each is a C₁₋₆ alkyl group, and more preferably, R₄ and R₅ are each methyl.

Ring A is a benzene ring optionally further having substituent(s) selected from a halogen atom, an optionally substituted hydrocarbon group, an optionally substituted hydroxy group and an optionally substituted amino group.

Ring A is preferably a benzene ring optionally further having 1 to 3 substituents selected from
a halogen atom;
a C₁₋₆ alkyl group optionally substituted with 1 to 3 C₆₋₁₄ aryloxy groups (preferably phenoxy);
a C₁₋₆ alkoxy group optionally substituted with 1 to 3 C₆₋₁₄ aryl groups (preferably phenyl); and
a C₆₋₁₄ aryloxy group (preferably phenoxy),
more preferably a benzene ring optionally further having 1 to 3 substituents selected from a halogen atom, a C₁₋₆ alkyl group and a C₁₋₆ alkoxy group, particularly preferably an unsubstituted benzene ring.

Ring B is a 5- to 7-membered ring.

As the "5- to 7-membered ring" for ring B, for example, 5- to 7-membered aromatic rings such as a benzene ring, a 5-to 7-membered aromatic heterocycle and the like; 5- to 7-membered non-aromatic rings such as a 5- to 7-membered alicyclic hydrocarbon, a 5- to 7-membered non-aromatic heterocycle and the like, can be mentioned.

As the 5- to 7-membered aromatic heterocycle, for example, a 5- to 7-membered monocyclic aromatic heterocycle containing, as a ring-constituting atom besides carbon atoms, 1 to 4 hetero atoms selected from an oxygen atom, a sulfur atom and a nitrogen atom can be mentioned.

As preferable examples of the monocyclic aromatic heterocycle, furan, thiophene, pyridine, pyrimidine, pyridazine, pyrazine, pyrrole, imidazole, pyrazole, isoxazole, isothiazole, oxazole, thiazole, oxadiazole, thiadiazole, triazole, tetrazole, triazine and the like can be mentioned.

As the 5- to 7-membered alicyclic hydrocarbon, a saturated or unsaturated alicyclic hydrocarbon having 5 to 7 carbon atoms, for example, a C₅₋₇ cycloalkane, a C₅₋₇ cycloalkene and the like can be mentioned.

As preferable examples of the C₅₋₇ cycloalkane, cyclopentane, cyclohexane, cycloheptane and the like can be mentioned.

As preferable examples of the C₅₋₇ cycloalkene, cyclopentene, cyclohexene, cycloheptene and the like can be mentioned.

As the 5- to 7-membered non-aromatic heterocycle, for example, a 5- to 7-membered monocyclic non-aromatic heterocycle containing, as a ring-constituting atom besides carbon atoms, 1 to 4 hetero atoms selected from an oxygen atom, a sulfur atom and a nitrogen atom can be mentioned.

As preferable examples of the monocyclic non-aromatic heterocycle, dihydrofuran, tetrahydrofuran, dihydrothiophene, tetrahydrothiophene, pyrrolidine, pyrroline, pyrazolidine, pyrazoline, piperidine, piperazine, morpholine, thiomorpholine, hexamethylenimine, oxazolidine, oxazoline, thiazolidine, thiazoline, imidazolidine, imidazoline, azepane, oxazepane, tetrahydropyridine, dihydropyridine and the like can be mentioned.

Ring B is preferably a 5- to 7-membered monocyclic non-aromatic heterocycle, more preferably tetrahydrofuran. That is, a ring represented by

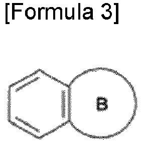

is

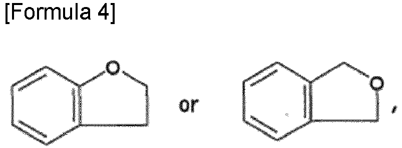

particularly preferably

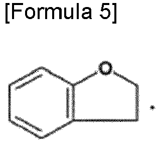

Y is a bond or CH₂. Y is preferably CH₂.

R is an optionally substituted hydroxy group. As used herein, the "substituent" which the "optionally substituted hydroxy group" optionally has is preferably a C₁₋₆ alkyl group.

R is preferably
a hydroxy group; or
a C₁₋₆ alkoxy group (preferably methoxy),
more preferably a hydroxy group.

In the formula (I), the partial structure:

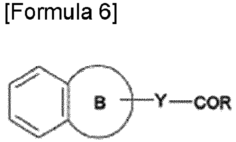

is preferably (2,3-dihydro-1-benzofuran-3-yl) acetic acid, namely

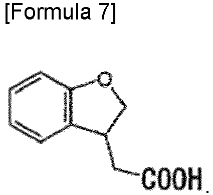

Especially, compound (I) having a partial structure of ((3S)-2,3-dihydro-1-benzofuran-3-yl) acetic acid has an excellent GPR40 receptor agonist activity, and is preferable.

As preferable examples of compound (I), the following compounds can be mentioned. Particularly, [(3S)-6-({2',6'-dimethyl-4'-[3-(methylsulfonyl)propoxy]biphenyl-3-yl}methoxy)-2,3-dihydro-1-benzofuran-3-yl] acetic acid (Fasiglifam), which is one of the Compounds C, is preferable as the GPR40 agonist in the present invention.

### [Compound A]

Compound (I) wherein
R¹ is a C₁₋₆ alkylsulfonyl group (preferably methylsulfonyl, ethylsulfonyl) or a 1,1-dioxidotetrahydrothiopyranyl group, each of which is optionally substituted with 1 to 3 substituents selected from a hydroxy group and the like [R¹ is preferably a C₁₋₆ alkylsulfonyl group (preferably methylsulfonyl, ethylsulfonyl), or a 1,1-dioxidotetrahydrothiopyranyl group optionally substituted with hydroxy group(s)];
X is a bond or a C₁₋₆ alkylene group (preferably a straight chain C₁₋₃ alkylene group);
R² and R³ are the same or different and each is
   a hydrogen atom;
   a halogen atom; or
   a C₁₋₆ alkyl group (preferably methyl);
R⁴ and R⁵ are the same or different and each is a C₁₋₆ alkyl group (preferably methyl);
ring A is a benzene ring optionally further having 1 to 3 substituents selected from a halogen atom, a C₁₋₆ alkyl group and a C₁₋₆ alkoxy group (preferably an unsubstituted benzene ring);
ring B is a 5- to 7-membered monocyclic non-aromatic heterocycle (preferably tetrahydrofuran);
Y is CH₂; and
R is a hydroxy group or a C₁₋₆ alkoxy group [R is preferably a hydroxy group].

### [Compound B]

Compound (I) wherein
R¹ is a C₁₋₆ alkylsulfonyl group (preferably methylsulfonyl, ethylsulfonyl) or a 1,1-dioxidotetrahydrothiopyranyl group, each of which is optionally substituted with 1 to 3 substituents selected from a hydroxy group and the like
[R¹ is preferably a C₁₋₆ alkylsulfonyl group (preferably methylsulfonyl, ethylsulfonyl), or a 1,1-dioxidotetrahydrothiopyranyl group optionally substituted with hydroxy group(s)];
   X is a bond or a C₁₋₆ alkylene group (preferably a straight chain C₁₋₃ alkylene group);
   R² and R³ are the same or different and each is
      a hydrogen atom;
      a halogen atom; or
      a C₁₋₆ alkyl group (preferably methyl);
   R⁴ and R⁵ are the same or different and each is a C₁₋₆ alkyl group (preferably methyl, ethyl) optionally substituted with hydroxy group(s)
[preferably, R⁴ and R⁵ are the same or different and each is a C₁₋₆ alkyl group (preferably methyl)];
   ring A is a benzene ring optionally further having 1 to 3 substituents selected from
      a halogen atom;
      a C₁₋₆ alkyl group optionally substituted with 1 to 3 C₆₋₁₄ aryloxy groups (preferably phenoxy);
      a C₁₋₆ alkoxy group optionally substituted with 1 to 3 C₆₋₁₄ aryl groups (preferably phenyl); and
      a C₆₋₁₄ aryloxy group (preferably phenoxy)
[ring A is preferably a benzene ring optionally further having 1 to 3 substituents selected from a halogen atom, a C₁₋₆ alkyl group and a C₁₋₆ alkoxy group, particularly preferably an unsubstituted benzene ring];
   ring B is a 5- to 7-membered monocyclic non-aromatic heterocycle (preferably tetrahydrofuran);
   Y is CH₂; and
   R is a hydroxy group or a C₁₋₆ alkoxy group
[R is preferably a hydroxy group].

### [Compound C]

Compound (I) which is selected from
[(3S)-6-({4'-[(4-hydroxy-1,1-dioxidotetrahydro-2H-thiopyran-4-yl)methoxy]-2',6'-dimethylbiphenyl-3-yl}methoxy)-2,3-dihydro-1-benzofuran-3-yl] acetic acid,
[(3S)-6-({2',6'-dimethyl-4'-[3-(methylsulfonyl)propoxy]biphenyl-3-yl}methoxy)-2,3-dihydro-1-benzofuran-3-yl] acetic acid,
[(3S)-6-({3'-fluoro-2',6'-dimethyl-4'-[3-(methylsulfonyl)propoxy]biphenyl-3-yl}methoxy)-2,3-dihydro-1-benzofuran-3-yl] acetic acid,
[(3S)-6-({3'-chloro-2',6'-dimethyl-4'-[3-(methylsulfonyl)propoxy]biphenyl-3-yl}methoxy)-2,3-dihydro-1-benzofuran-3-yl] acetic acid,
[(3S)-6-({3',5'-dichloro-2',6'-dimethyl-4'-[3-(methylsulfonyl)propoxy]biphenyl-3-yl}methoxy)-2,3-dihydro-1-benzofuran-3-yl] acetic acid, and
[(3S)-6-({2',6'-diethyl-4'-[3-(methylsulfonyl)propoxy]biphenyl-3-yl}methoxy)-2,3-dihydro-1-benzofuran-3-yl] acetic acid).

As a salt of compound (I), for example, metal salts, an ammonium salt, salts with organic bases, salts with inorganic acids, salts with organic acids, salts with basic or acidic amino acids and the like can be mentioned.

Preferable examples of the metal salt include alkali metal salts such as sodium salt, potassium salt and the like; alkaline earth metal salts such as calcium salt, magnesium salt, barium salt and the like; aluminum salt and the like.

Preferable examples of the salt with organic base include a salt with trimethylamine, triethylamine, pyridine, picoline, 2, 6-lutidine, ethanolamine, diethanolamine, triethanolamine, cyclohexylamine, dicyclohexylamine, N,N'-dibenzylethylenediamine and the like.

Preferable examples of the salt with inorganic acid include a salt with hydrochloric acid, hydrobromic acid, nitric acid, sulfuric acid, phosphoric acid and the like.

Preferable examples of the salt with organic acid include a salt with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, α-toluenesulfonic acid and the like.

Preferable examples of the salt with basic amino acid include a salt with arginine, lysine, ornithine and the like. Preferable examples of the salt with acidic amino acid include a salt with aspartic acid, glutamic acid and the like.

Of the above-mentioned salts, a pharmacologically acceptable salt is preferable.

The prodrug of the compound (I) is a compound which is converted to the compound (I) with a reaction due to an enzyme, gastric acid, etc. under the physiological condition in the living body, that is, a compound which is converted to the compound (I) by enzymatic oxidation, reduction, hydrolysis, etc.; a compound which is converted to the compound (I) by hydrolysis etc. due to gastric acid, and the like.

Examples of a prodrug of compound (I) include a compound wherein an amino group of compound (I) is acylated, alkylated or phosphorylated (e.g., a compound wherein an amino group of compound (I) is eicosanoylated, alanylated, pentylaminocarbonylated, (5-methyl-2-oxo-I, 3-dioxolen-4- yl) luethoxycarbonylated, tetrahydrofuranylated, pyrrolidylmethylated, pivaloyloxymethylated or tert-butylated); a compound wherein a hydroxy group of compound (I) is acylated, alkylated, phosphorylated or borated (e.g., a compound wherein a hydroxy group of compound (I) is acetylated, palmitoylated, propanoylated, pivaloylated, succinylated, fumarylated, alanylated or dimethylaminomethylcarbonylated); a compound wherein a carboxyl group of compound (I) is esterified or amidated (e.g., a compound wherein a carboxyl group of compound (I) is -C₁₋₆ alkyl esterified, phenyl esterified, carboxymethyl esterified, dimethylaminomethyl esterified, pivaloyloxymethyl esterified, ethoxycarbonyloxyethyl esterified, phthalidyl esterified, (5-methyl-2-oxo-I, 3-dioxolen-4-yl)methyl esterified, cyclohexyloxycarbonylethyl esterified or methylamidated) and the like. Of these, a compound wherein a carboxyl group of compound (I) is esterified by C₁₋₆ alkyl group such as methyl, ethyl, tert-butyl and the like is preferable. These compounds can be produced from compound (I) according to a method known per se.

A prodrug of the compound (I) may be a compound that converts to the compound (I) under physiological conditions as described in Development of Pharmaceutical Products, vol. 7, Molecule Design, 163-198, Hirokawa Shoten (1990).

When compound (I) includes stereoisomers, both the isomers alone and mixtures of each isomers are included in the scope of the present invention.

In addition, compound (I) may be a hydrate or non-hydrate. A hydrate of compound (I) normally shows an excellent preservation stability.

Compound (I) may be labeled with an isotope (e.g., ³H, ¹⁴C, ³⁵S) or the like.

Compound (I) or a salt thereof, or a prodrug of compound (I) are suitable for use as an agonist of GPR40, which is the first component of the pharmaceutical composition of the present invention, because they have less toxicity (e.g., less impacts on hematologic parameters such as a red blood cell count, a hematocrit value, a hemoglobin concentration, MCH, MCHC, MCV, a platelet count, a white blood cell count, a blood reticulocyte count, and white blood cell classification; and blood biochemical parameters such as total protein, albumin, an A/G ratio, glucose, total cholesterol, triglycerides, urea nitrogen, creatinine, total bilirubin, AST, ALT, LDH, ALP, CK, Na, K, CI, calcium, inorganic phosphorus, and retinol (vitamin A)), and less side effects (e.g., acute toxicity, chronic toxicity, genotoxicity, reproductive toxicity, cardiotoxicity, drug interaction, and carcinogenicity).

### - Second component: a fatty acid that is an endogenous ligand of GPR40

Various fatty acids are known as the "fatty acid that is an endogenous ligand of GPR40". The "fatty acid that is an endogenous ligand of GPR40" which can be used as the second component of the pharmaceutical composition of the present invention, is not particularly limited and can be selected from those known in the art in consideration of the action and effect of the present invention, which is typically a medium- or long-chain saturated or unsaturated fatty acid (having about 6 to 22 carbon atoms), for example, a saturated fatty acid having 12 to 16 carbon atoms and an unsaturated fatty acid having 18 to 20 carbon atoms. In one embodiment of the present invention, the second component is preferably an unsaturated fatty acid having 18 to 20 carbon atoms, such as γ-linolenic acid and docosahexaenoic acid. The position of an unsaturated bond (double bond) in the unsaturated fatty acid may be n-3 (ω3), n-6 (ω6), n-9 (ω9) or any other position, and the number of unsaturated bonds (double bonds) may be one, two, three, four, five or six. As the second component, any one kind of the fatty acid that is an endogenous ligand of GPR40 may be used alone, or two or more kinds of the fatty acid may be used in combination.

Specific examples of the medium- or long-chain saturated fatty acid include caproic acid (hexanoic acid, 6:0), caprylic acid (octanoic acid, 8:0), pelargonic acid (nonanoic acid, 9:0), capric acid (decanoic acid, 10:0), lauric acid (dodecanoic acid, 12:0), myristic acid (tetradecanoic acid, 14:0), pentadecylic acid (pentadecanoic acid, 15:0), palmitic acid (hexadecanoic acid, 16:0), margaric acid (heptadecanoic acid, 17:0), stearic acid (octadecanoic acid, 18:0), arachidic acid (icosanoic acid, 20:0), heneicosylic acid (21:0), and behenic acid (docosanoic acid, 22:0).

Specific examples of the medium- or long-chain unsaturated fatty acid include myristoleic acid (14:1, n-5), palmitoleic acid (16:1, n-7), sabienic acid (16:1, n-10), oleic acid (18:1, n-9, cis), elaidic acid (18:1, n-9, trans), vaccenic acid (18:1, n-7), gadoleic acid (20:1, n-11), eicosenoic acid (20:1, n-9), erucic acid (22:1, n-9); linoleic acid (18:2, n-6), eicosadienoic acid (20:2, n-6), docosadienoic acid (22:2, n-6); α-linolenic acid (18:3, n-3), γ-linolenic acid (18:3, n-6), pinolenic acid (18:3, n-6), α-eleostearic acid (18:3, n-5), β-eieostearic acid (18:3, n-5), mead acid (20:3, n-9), dihomo-γ-linolenic acid (20:3, n-6), eicosatri hydrochloric acid (20:3, n-3); stearidonic acid (18:4, n-3), arachidonic acid (20:4, n-6), eicosatetraenoic acid (20:4, n-6), adrenic acid (22:4, n-6); bosseopentaenoic acid (18:5, n-4), eicosapentaenoic acid (20:5, n-3), osbond acid (22:5, n-6), clupanodonic acid (22:5, n-3), and docosahexaenoic acid (22:6, n-3).

### Use of the pharmaceutical composition (the pharmaceutical composition for preventing or treating a bone disease)

The pharmaceutical composition of the present invention can be used for preventing or treating a bone disease. The "bone disease" is, to be more specific, a disease resulting (at least in part) from bone resorption exceeding bone formation because of an increase in activity of or formation (differentiation) into osteoclasts, which can be prevented or treated by suppressing the increase. Examples of such a bone disease include diseases, symptoms or conditions such as osteoporosis (postmenopausal osteoporosis, senile osteoporosis, secondary osteoporosis due to the use of a therapeutic agent for a steroid or hormonal therapy or the like, and rheumatoid arthritis-associated osteoporosis), rheumatoid arthritis-associated bone destruction, cancerous hypercalcemia, bone destruction due to multiple myelomas and bone metastasis of cancers, giant cell tumor, osteopenia, periprosthetic osteolysis, bone destruction in chronic osteomyelitis, Paget's disease of bone, renal osteodystrophy, osteogenesis imperfecta, alveolar bone resorption due to periodontal diseases (such as periodontitis), alveolar bone resorption after tooth extraction, and alveolar bone resorption after alveolar bone generation. For example, alveolar bone resorption due to a periodontal disease (such as periodontitis), alveolar bone resorption after tooth extraction, bone resorption after alveolar bone generation, and other diseases, symptoms or conditions accompanied by alveolar bone resorption (referred to herein as "alveolar bone resorption-related diseases etc.") are bone diseases preferable as a target for prevention or treatment with the pharmaceutical composition of the present invention.

As used herein, the term "treatment/treating" means, in addition to complete curing of a diseased condition, suppressing the progress and/or deterioration of symptoms even if the diseased condition is not completely cured, thereby stopping the progress of the diseased condition, or improving part or all of the diseased condition to lead toward a cure, and the term "prevention/preventing" means preventing, suppressing or delaying of the onset of the diseased condition.

The pharmaceutical composition (a pharmaceutical composition) of the present invention can be a product exhibiting a therapeutic or preventive effect as described above, for example, a product according to the classification such as a drug, or a quasi-drug (a cosmeceutical), which is an intermediate between a drug and a cosmetic, in accordance with the legal system of each country.

The pharmaceutical composition of the present invention is formulated in an appropriate dosage form according to a bone disease to be prevented or treated, in accordance with known or well-known conventional techniques. Examples of the dosage form for the pharmaceutical composition of the present invention include oral preparations such as tablets (including a sublingual tablet and an oral disintegration tablet), capsules (including a soft capsule and a microcapsule), granules, powders, troches, syrups, emulsions, and suspensions; and parenteral preparations such as injections (e.g., a subcutaneous injection, an intravenous injection, an intramuscular injection, an intraperitoneal injection, an intravenous infusion), external preparations (e.g., a transdermal preparation, an external solid preparation, an external solution, a spray, an ointment, a cream, a gel, a transdermal patch), suppositories (e.g., a rectal suppository and a vaginal suppository), pellets, nasal preparations, pulmonary preparations (inhalants), and eye drops. These preparations may be controlled-release preparations (e.g., sustained-release microcapsules), such as an immediate-release preparation or a sustained-release preparation.

In a preferred embodiment of the present invention, the pharmaceutical composition of the present invention is formulated as that for preventing or treating an alveolar bone resorption-related disease such as a periodontal disease. Such a medial composition is preferably prepared as a dosage form suitable for topical administration to a site in an oral cavity where the alveolar bone resorption-related disease has occurred or is likely to occur, for example, as an external preparation (including mouthwash, toothpaste, and tooth powder), a tablet, a troche, or an injection. For example, when the pharmaceutical composition of the present invention is prepared as an external preparation, apatite or the like may be used as a base (matrix), and each active ingredient may be mixed therein to form a paste, or a polymeric compound such as hydroxypropyl cellulose may be used as a base, and each active ingredient may be mixed therein to form a viscous external solution. The pharmaceutical composition for preventing or treating an alveolar bone resorption-related disease such as a periodontal disease according to the present invention may be sold as a commercial product, or may be used in a medical institution or the like in a periodontal disease-related surgery or treatment (periodontal tissue regeneration therapy, light-activated disinfection therapy, maintenance, etc.).

The pharmaceutical composition of the present invention can be directed to bone diseases in mammals. The mammals encompass humans and mammals except for humans (non-human mammals) such as mice, rats, hamsters, guinea pigs, rabbits, dogs, cats, pigs, cows, horses, sheep, monkeys, and the like.

The pharmaceutical composition of the present invention may contain, in addition to the first and second components, a further active ingredient that is effective for preventing or treating the bone disease, as necessary. In other words, in the pharmaceutical composition of the present invention, the first and the second components can be used in combination with a known therapeutic drug (component) for the bone disease.

Examples of the known therapeutic drug for bone diseases can include bisphosphonates (e.g., alendronate, etidronate, ibandronate, incadronate, pamidronate, risedronate, zoledronate), active vitamin D3, calcitonin and derivatives thereof, hormones such as estradiol, SERMs (selective estrogen receptor modulators), ipriflavone, vitamin K2 (menatetrenone), calcium preparations, PTH (parathyroid hormone), nonsteroidal anti-inflammatory agents (e.g., celecoxib, rofecoxib), soluble TNF receptors (e.g., etanercept), anti-TNF α antibodies or antigen-binding fragments thereof (e.g., infliximab), anti-PTHrP (parathyroid hormonerelated protein) antibodies or antigen-binding fragments thereof, IL-1 receptor antagonists (e.g., anakinra), anti-IL-6 receptor antibodies or antigen-binding fragments thereof (e.g., tocilizumab), anti-RANKL antibodies or antigen-binding fragments thereof (e.g., denosumab), and OCIF (osteoclastogenesis inhibitory factor).

For example, when the pharmaceutical composition of the present invention is prepared as a pharmaceutical composition for preventing or treating an alveolar bone resorption-related disease, it is possible to use a known component effective for preventing or treating periodontal diseases and the like (a therapeutic agent for periodontal diseases) as the aforementioned further component in combination with the first component and the second component of the present invention. Examples of the therapeutic agent for periodontal diseases include antibacterial agents (amoxycillin, augmentin, tetracycline, minocycline, clarithromycin, levoflocaxin, clindamycin, metronidazole and the like), anti-inflammatory agents (tranexamic acid, epsilon-aminocaproic acid, azulene, allatoin, aluminum chlorohydroxy allantoinate, aluminum dihydroxy allantoinate, glycyrrhetinic acid salts, glycyrrhetinic acids, dihydrocholesterol, hydrocholesterol and the like), bactericides (cetylpyridinium chloride, isopropyl methylphenol, sodium hypochlorite, polyethylene glycol, lauroylsarcosine salts, hinokitiol, and the like), blood circulation accelerators (tocopherol nicotinate, pyridoxine hydrochloride, tocopherol acetate, and the like), periodontal tissue regenerating agents (Trafermin preparations) "REGROTH" (registered trademark, KAKEN PHARMACEUTICAL CO., LTD.), and photosensitive gels.

The pharmaceutical composition of the present invention is usually prepared as a pharmaceutical composition (preparation) containing the first and second components (and a further active ingredient other than the first and second components, as necessary), a pharmacologically acceptable base (carrier, matrix) and an additional additive, as necessary, in accordance with known or well-known conventional techniques. The amounts of various base materials and additives blended in the pharmaceutical composition can be appropriately adjusted in the present invention as well in accordance with known or well-known conventional pharmaceutical compositions.

In the present invention, a pharmacologically acceptable carrier generally used in pharmaceutical compositions in various dosage forms can be used. For example, excipient, lubricant, binder and disintegrant for solid preparations; solvent, dissolution aids, suspending agent, isotonicity agent, buffer and soothing agent for liquid preparations, and abrasive, humectant, binder and surfactant for paste preparations can be mentioned.

Examples of the excipient include lactose, sucrose, D-mannitol, starch, corn starch, crystalline cellulose and light anhydrous silicic acid.

Examples of the lubricant include magnesium stearate, calcium stearate, talc and colloidal silica.

Examples of the binder include crystalline cellulose, sucrose, D-mannitol, dextrin, hydroxypropylcellulose, hydroxypropylmethylcellulose, polyvinylpyrrolidone, starch, saccharose, gelatin, methylcellulose and carboxymethylcellulose sodium.

Examples of the disintegrant include starch, carboxymethylcellulose, carboxymethylcellulose calcium, carboxymethylstarch sodium and L-hydroxypropylcellulose.

Examples of the solvent include water for injection, alcohol, propylene glycol, macrogol, sesame oil, corn oil and olive oil.

Examples of the dissolution aids include polyethylene glycol, propylene glycol, D-mannitol, benzyl benzoate, ethanol, trisaminomethane, cholesterol, triethanolamine, sodium carbonate and sodium citrate.

Examples of the suspending agent include surfactants such as stearyltriethanolamine, sodium lauryl sulfate, lauryl aminopropionate, lecithin, benzalkonium chloride, benzethonium chloride and glycerol monostearate; and hydrophilic polymers such as polyvinyl alcohol, polyvinylpyrrolidone, carboxymethylcellulose sodium, methylcellulose, hydroxymethylcellulose, hydroxyethylcellulose and hydroxypropylcellulose.

Examples of the isotonicity agent include glucose, D-sorbitol, sodium chloride, glycerin and D-mannitol.

Examples of the buffer include buffers of phosphates, acetates, carbonates or citrates.

Examples of the soothing agent include benzyl alcohol.

Examples of the abrasive include precipitated silica, silica gel, aluminosilicate, zeolite, zirconosilicate, dibasic calcium phosphate dihydrate and anhydrous, calcium pyrophosphate, calcium carbonate, aluminum hydroxide, alumina, magnesium carbonate, tribasic magnesium phosphate, insoluble sodium metaphosphate, insoluble potassium metaphosphate, titanium oxide, hydroxyapatite, and synthetic resinous abrasives.

Examples of the humectant include glycerin, sorbitol, propyleneglycol, polyethyleneglycol, 1,3-butylene glycol, xylitol, maltitol, lactitol, trehalose, and Tornare.

Examples of the binder include carrageenan, sodium carboxymethylcellulose, sodium alginate, polyacrylic acid, sodium polyacrylate, carboxyvinyl polymer, polyvinyl alcohol, xanthan gum, tara gum, guar gum, locust bean gum, gellan gum, gelatin, curdlan, gum arabic, agar, and pectin.

Examples of the surfactant include an anionic surfactant, a cationic surfactant, an amphoteric surfactant, and a non-ionic surfactant. Examples of the anionic surfactant include sodium lauryl sulfate, sodium lauroyl sarcosine, methyl lauroyl taurate, acyl amino acid salts, sodium dodecylbenzenesulfonate, sodium α-sulfofatty acid alkyl ester, and alkyl phosphate ester salts. Examples of the cationic surfactant include alkylammonium, and alkylbenzylammonium salts. Examples of the amphoteric surfactant include betaine acetate amphoteric surfactants such as an alkyl dimethyl amino acetic acid betaine and a fatty acid amido propyl dimethyl amino acetic acid betaine, imidazoline amphoteric surfactants such as an N-fatty acid acyl-N-carboxymethyl-N-hydroxyethyl ethylenediamine salt, and amino acid surfactants such as an N-fatty acid acyl-L-alginate salt. Examples of the non-ionic surfactant include polyglycerol fatty acid ester, polyoxyethylene alkyl ether, polyoxyethylene hydrogenated castor oil, polyoxyethylene sorbitan fatty acid ester, sucrose fatty acid ester, sorbitan fatty acid ester, polyoxyethylene polyoxypropylene block copolymer activator, polyoxyethylene fatty acid ester, and fatty acid monoglyceride.

For the additive used as necessary, an additive commonly used in pharmaceutical compositions in various dosage forms, for example, a preservative, an antioxidant, a colorant, a sweetening agent, an adsorbent, or a humectant, can be used in the present invention.

Examples of the preservative include parahydroxybenzoic acid esters, chlorobutanol, benzyl alcohol, phenethyl alcohol, dehydroacetic acid, and sorbic acid.

Examples of the antioxidant include sulfite, ascorbic acid, and α-tocopherol.

Examples of the colorant include water-soluble tar-based food colorings (e.g., food colorings such as Food Red Nos. 2 and 3, Food Yellow Nos. 4 and 5, Food Blue Nos. 1 and 2), water-insoluble lake pigments (e.g., an aluminum salt of the water-soluble tar-based food coloring), and natural pigments (e.g., β-carotene, chlorophyll, red iron oxide).

Examples of the sweetening agent include sodium saccharin, dipotassium glycyrrhizinate, aspartame, and stevia.

The daily dose of the pharmaceutical composition of the present invention (each of the first component, the second component, and an additional active ingredient used as necessary) varies depending on the type of bone disease, the degree of symptoms; the animal species, age, sex, body weight, and difference in sensitivity of the administration subject; the method, route, time, interval, the dosage form of the pharmaceutical composition in the administration; the type of the active ingredient, and the like, and is not particularly limited. For example, when the pharmaceutical composition of the present invention is prepared as that for preventing or treating a bone disease in humans, a person skilled in the art would be able to determine, without undue experimentation, an administration method or daily dose appropriate for administration to humans and evaluate the efficacy and safety of the prevention or the treatment through experiments using an animal model of the bone disease or clinical trials for humans.

In one embodiment of the present invention, the pharmaceutical composition of the present invention exhibits the action and effect of suppressing differentiation of a hematopoietic stem cell into an osteoclast in vitro. In such an embodiment, the concentration of the first component (GPR40 agonist) in the medium can be, for example, in the range of from 0.1 to 100 µM, and the concentration of the second component (endogenous ligand of GPR40) in the medium can be, for example, in the range of from 0.1 to 1000 µM. The suppression of the differentiation into an osteoclast can be examined, for example, by a decrease in expression level of an osteoclast marker gene (at least one of Ctsk, Calcr, TRAP, or the like) in the cultured cells. When the pharmaceutical composition of the present invention is formulated, the contents of the first component and the second component in the pharmaceutical composition and the daily dose can be adjusted in consideration of the above-described concentrations in the medium.

The pharmaceutical composition of the present invention contains at least the first component and the second component, and may further contain an additional active ingredient as necessary, and these components may be contained in a single preparation or in separate (two or more) preparations. That is, the pharmaceutical composition of the present invention may be either of the ones in which (i) each active ingredient is administered simultaneously through the same administration route by a single preparation, (ii) each active ingredient is administered simultaneously through the same administration route by a plurality of preparations, (iii) each active ingredient is administered with a time difference through the same administration route by a plurality of preparations, (iv) each active ingredient is administered simultaneously through a plurality of administration routes by a plurality of preparations, and (v) each active ingredient is administered with a time difference through a plurality of administration routes by a plurality of preparations.

### -Method and use-

A method for preventing or treating a bone disease in a mammal of the present invention includes administering to the mammal an effective dose of a GPR40 agonist and an effective dose of a free fatty acid that is an endogenous ligand of GPR40.

Use of a GPR40 agonist and a free fatty acid that is an endogenous ligand of GPR40 of the present invention is for the production of a pharmaceutical composition for preventing or treating a bone disease.

The technical matters described herein in relation to the pharmaceutical composition of the present invention are applicable similarly in relation to the above-described method and use according to the present invention.

The pharmaceutical composition, method, and use of the present invention may be further converted into an invention with another aspect based on the description herein. For example, in one aspect, the present invention provides a method for suppressing differentiation into an osteoclast and/or a method for promoting differentiation into an osteoblast, using a GPR40 agonist and a free fatty acid that is an endogenous ligand of GPR40 (including, for example, a step of contacting the GPR40 agonist and the free fatty acid with a cell in vivo or in vitro).

### Examples

In the following examples, Fasiglifam was prepared by the method described in Patent Literature 1 (WO2008/001931) as cited above.

### [Test Example 1] Inhibitory effect on osteoclast differentiation of mouse macrophage-derived cells

The differentiation into osteoclasts is induced when osteoclast precursors are stimulated by a receptor activation of nuclear factor-kB ligand (RANKL), which is produced by an osteoblast or the like. In macrophage cells that were osteoclast precursors, the inhibitory effect on the osteoclast differentiation was examined in the treatment with a compound in the presence of RANKL.

R264.7 cells purchased from KAC Co., Ltd. were used in the test. The cells were suspended in DMEM with 10% serum, seeded in a 96-well plate at a concentration of 6000 cells/well, and cultured at 37°C under a 5% CO₂ condition. After an overnight culture, the medium was replaced with differentiation medium (50 ng/mL RANKL, DMEM including 10% serum), and simultaneously, a free fatty acid at 10 µM and Fasiglifam at 10 µM were added alone or in combination. γ-linolenic acid (SIGMA) or docosahexaenoic acid (SIGMA) was used as the free fatty acid. The medium exchange and the compound addition were performed every other day, the medium was removed on day 3 of the culture with the differentiation medium, a cytolytic solution (buffer RLT, RNeasy, QIAGEN) was added and mixed for 5 minutes, and samples were collected. RNA was extracted from each sample using RNeasy 96 kit (QIAGEN) and a total RNA was obtained after a DNase treatment. The obtained total RNA was reverse-transcribed into cDNA using SuperScript IV (Thermo Fisher), and then the expression of the osteoclast marker genes (Ctsk, Calcr) was measured by quantitative PCR (ViiA7, Applied Biosystems). TaqMan Fast Advanced Master Mix (Applied Biosystems) was used for the PCR buffer, and TaqMan Gene Expression Assays (Applied Biosystems) were used for the primer and probe complementary to each gene. The expression level of each gene was adjusted with the expression level of β-actin and shown as a relative value when the expression level of the RANKL non-added group was set to 1.0.

The results were as shown in Figure 1. It was confirmed that the use of the saturated fatty acid and Fasiglifam in combination enhanced the inhibitory effect on osteoclast differentiation as compared with the treatment with the saturated fatty acid alone.

## Claims

1. A pharmaceutical composition for preventing or treating a bone disease, comprising a GPR40 agonist and a free fatty acid that is an endogenous ligand of GPR40.

2. The pharmaceutical composition according to claim 1, wherein the GPR40 agonist is a compound represented by formula (I) or a salt thereof, or a prodrug of the compound represented by formula (I) 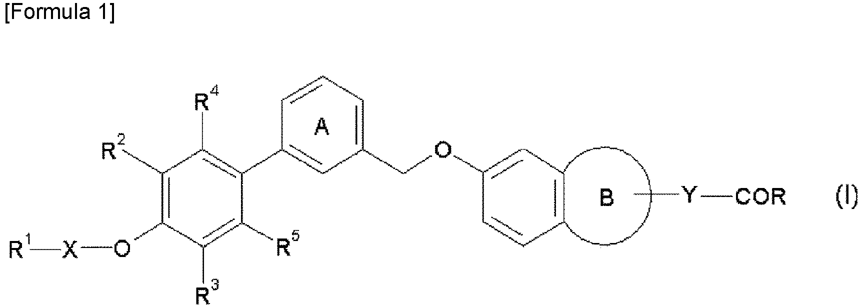 wherein
R¹ represents R⁶-SO₂- (R⁶ represents a substituent) or an optionally substituted 1,1-dioxide tetrahydrothiopyranyl group;
X represents a bond or a divalent hydrocarbon group;
R² and R³ each represent a hydrogen atom, a halogen atom, an optionally substituted hydrocarbon group or an optionally substituted hydroxy group, and are optionally the same or different;
R⁴ and R⁵ each represent a C₁-₆ alkyl group optionally substituted with a hydroxy group, and are optionally the same or different;
ring A represents a benzene ring that optionally further includes a substituent selected from the group consisting of a halogen atom, an optionally substituted hydrocarbon group, an optionally substituted hydroxy group, and an optionally substituted amino group;
ring B represents a 5- to 7-membered ring;
Y represents a bond or CH₂; and
R represents an optionally substituted hydroxy group.

3. The pharmaceutical composition according to claim 1, wherein the free fatty acid that is the endogenous ligand of GPR40 is one or more saturated or unsaturated fatty acids each having 6 to 22 carbon atoms.

4. The pharmaceutical composition according to claim 1, wherein the bone disease is alveolar bone resorption in a periodontal disease, alveolar bone resorption after a tooth extraction, bone resorption after alveolar bone reconstruction surgery, or another disease, symptom or condition accompanied by alveolar bone resorption.

5. A method for preventing or treating a bone disease in a mammal, comprising administering to the mammal an effective dose of a GPR40 agonist and an effective dose of a free fatty acid that is an endogenous ligand of GPR40.

6. Use of a GPR40 agonist and a free fatty acid that is an endogenous ligand of GPR40 for producing a pharmaceutical composition for preventing or treating a bone disease.
